# EUROPEAN PATENT SPECIFICATION

(11) **EP 2 740 463 B1**
(45) Date of publication and mention of the grant of the patent: **09.11.2016**
(21) Application number: 12817100.6
(22) Date of filing: 27.07.2012
(51) Int. Cl.: A61K 8/14, A61K 8/96, A61K 35/00, A61K 35/60, C07K 14/435

(54) **USES OF MEMBRANE VESICLES OF A MARINE ORGANISM FOR THE INDUSTRIAL USE THEREOF AND METHOD FOR OBTAINING SAME**
VERWENDUNG VON MEMBRANVESIKELN AUS EINEM MARINEN ORGANISMUS ZUR INDUSTRIELLEN VERWENDUNG UND VERFAHREN ZUR HERSTELLUNG
PROCÉDÉS D'OBTENTION ET UTILISATIONS DE VÉSICULES DE MEMBRANE D'UN ORGANISME MARIN POUR UNE UTILISATION INDUSTRIELLE

(30) Priority: 28.07.2011 ES 201131299 P
(43) Date of publication of application: 11.06.2014
(73) Proprietor: Consejo Superior De Investigaciones Científicas (CSIC), 28006 Madrid (ES)
(72) Inventor: CARVAJAL ALCARAZ, Micaela, E-30100 Espinardo (Murcia) (ES); GARCÍA VIGUERA, Cristina, E-30100 Espinardo (Murcia) (ES); MORENO FERNÁNDEZ, Diego Ángel, E-30100 Espinardo (Murcia) (ES); MARTÍNEZ BALLESTA, María del Carmen, E-30100 Espinardo (Murcia) (ES)
(74) Representative: Ungria López, Javier
(86) International application number: PCT/ES2012/070579
(87) International publication number: WO 2013/014325

(56) References cited:
- WO-A1-2012/160232
- JP-A- 2010 018 575
- US-A1- 2006 099 273
- US-B1- 6 899 863
- Anonymous: "Medusas del Mar Menor contra la deshidratación de la piel", Murciaeconomia , 20 June 2014 (2014-06-20), XP002731068, Retrieved from the Internet: URL:http://murciaeconomia.com/not/26391/me dusas-del-mar-menor-contra-la-deshidrataci on-de-la-piel/ [retrieved on 2014-10-14]
- HARTMANN, A ET AL.: 'Synaptic membranes from Torpedo marmorata electric organ. 1. Separation and analysis of nicotinic acetylcholine receptor and acetylcholinesterase-containing membrane vesicles using aqueous two-phase systems.' BIOCHEMICA ET BIOPHYSICA ACTA vol. 513, 1978, pages 382 - 394, XP055118557
- BUCK, M ET AL.: 'Driving forces for the uphill transport of amino acids into epidermal brush border membrane vesicles of the sea anemone, Anemona sulcata (Cnidaria, Anthozoa).' COMP. BIOCHEM. PHYSIOL. vol. 88A, no. 2, 1987, pages 273 - 279, XP023585151
- MULLER, W.E.G ET AL.: 'The multixenobiotic resistance mechanism in the marine sponge Suberites domuncula: its potential applicability for the evaluation of enviromental pollution by toxic compounds.' MARINE BIOLOGY vol. 125, 1996, pages 165 - 170, XP008169697
- KURELEC, B ET AL.: 'Evidence for a multixenobiotic resistance mechanism in the mussel Mytilus galloprovincialis.' AQUATIC TOXICOLOGY vol. 19, 1991, pages 291 - 302, XP023772800
- CUCUSLESCU, M ET AL.: 'The isolation of a plasma membrane-rich fraction from the skeletal muscle of two species of marine crab, Carcinus maenas and Cancer pagurus. Comp.' COMP. BIOCHEM. PHYSIOL. vol. 106B, no. 2, 1993, pages 263 - 267, XP023527471
- MORRÉ D. J ET AL.: 'Preparation of mammalian plasma membranes by aqueous two-phase partition. Cell membrane technique- part 2.' BIOTECHNIQUES vol. 7, no. 9, 1989, pages 946 - 958, XP008169699
- UEHARA, S ET AL.: 'Isolation of neuronal plasma membranes from the crayfish Procamburus clarkii, with an aqueous two phase polymer system followed by sucrose density gradient centrifugation.' BIOCHIMICA ET BIOPHYSICA ACTA vol. 556, no. 1, 1979, pages 96 - 104, XP023364415

## Description

### FIELD OF THE ART

The present invention relates to the fields of chemistry and pharmacy, and specifically refers to membrane vesicles of natural origin, specifically derived from a marine animal, which have high content of intrinsic membrane proteins for dermatologic, pharmacological or therapeutic purposes.

### STATE OF THE ART

### Importance of membrane proteins

In animal cells, membrane proteins (MP) represent a strategic point to a potential therapeutic intervention, being the MP targets for drugs in cancer research, for example (Harvey et al. Physiol. Genomics 2001, 5, 129-136). In animal cells, the MP controls many primary functions such as ion and metabolite transport, endocytosis, cell proliferation, etc. All of these processes involve a wide range of highly variable protein in terms of structure and function. This large variability in the nature of membrane proteins lead to the number of extraction processes are at the same time both extensive and specific. Thus, besides the wide range of molecular weights and isoelectric points of proteins, they differ in their hydrophobic nature, in the case of intrinsic proteins. Also, they differ in the variability of surrounding lipid content. All this make them not always easy to remove and purify, especially proteins with multiple transmembrane domains embedded in the lipid phase of the membrane.

Therefore, the use of membrane vesicles containing a protein of interest able of maintaining their functionality and properties can be an additional advantage in terms of stability and the extraction economic cost.

The movements of almost all solutes through the membrane are mediated by membrane transport proteins, more or less specialized in transport of specific molecules. Since diversity and physiology of different cells of an organism is related largely to its ability to transport outside molecules, it is postulated that there must be a pool of specific transport proteins for each cell type and for each physiological stage (Lodish et al, 2005, cell and Molecular Biology, Buenos Aires. Medica Panamericana; ISBN 950-06-1974-3).This differential expression is regulated by: the differential transcription of the genes coding for these proteins and their translation.

These transmembrane proteins have got multitude of α-helices embedded in the lipid matrix. This structure is likely to involve a pathway through protein hydrophilic environment that would cause a disruption in the medium consisting of highly hydrophobic lipids (Lodish et al, 2005, Cell and Molecular Biology, Buenos Aires. Panamericana Medical, ISBN 950-06 -1974 to 3). The proteins involved various forms of transport such as, ATP- pumps, (energy is needed), or facilitated diffusion channels.

Some of these proteins are able of transporting water, ions and small solutes through them, such as glycerol and urea (both hygroscopic agents widely used in cosmetics).

An additional problem in the extraction of integral membrane proteins is that they are minor constituents compared to other soluble cell proteins. To date, it has been possible to extract these functional proteins when they are inserted into the lipid bilayer.

It has been possible to obtain hydrophobic proteins from membrane fractions with reduced complexity and acceptable yield (higher than 1%, referred to the obtained membrane fraction from the total weight of protein in the crude homogenate) by membrane extraction method employed in shellfish by several authors (Cuculescu and Bowler, 1993 Comp. Biochem. Physiol Vol. 10611, No. 2, p. 263-267, 1993). However, by this method, vesicles are obtained from manual tissue homogenization requiring sucrose/Percoll gradient, which is not extrapolated to industrial process due to expensive agents used, such as histidine, phenylmethylsulfonyl fluoride (PMSF) and triphosphate adenosine (ATP). Furthermore, it requires a processing of the starting material to remove shell or exoskeleton that protects the soft tissue of the animal, which is then used in the homogenization process to obtain vesicles.

Liposomes have been considered for long time most innovative contribution in dermatology, with pharmaceutical and cosmetic purposes. However, due to their high cost, variable phospholipid purity and unstable nature, the surfactants containing vesicles are an advantageous alternative. Furthermore, these vesicles contain membrane intrinsic proteins capable of acting as carriers of substances.

### Importance of substances from marine organisms

The study of marine organisms has increased in recent years due to their bioactive potential. Many marine species, from sharks to algae produce bioactive compounds with important applications in the medical field (Amador et al, Progress in the development and acquisition of anticancer agents from marine sources. Ann Oncol 2003. 14, 1607-1615; Calvert, Kelp Beds as Fish and Invertebrate Habitat in Southeastern Alaska, Masters Thesis 2005, University of Alaska, Fairbanks, p 113; Fusetani N., 2000, Introduction in: N. Fusetani, Editor, Drugs from the Sea, Karger, Basel (2000), pp. 1-5; Blunt et al., Marine Natural Products 2008 Nat Prod Rep , 25.35 to 94).

In the Mediterranean Sea, most of bioactive molecules (antibacterial, antifungal, antiviral, cytotoxic) have been isolated from species such as algae or phanerogams and particularly animals such as sponges, echinoderms, polychaetes, ascidians, molusks, bryozoans and cnidarians ( Ballesteros et al., Biological activity of extract from some Mediterranean macrophytes Botanica Marina, 1995 , 35, 481-485; Calf et al, Multiple functions for secondary metabolites in encrusting marine invertebrates J. Chem Ecol 1997 , 23, 1527-1547).

Thus, proteins from marine organisms have multiple applications. For example, they are getting bioluminescent molecules, such as luciferin, from organisms of the genus Cnidaria mainly, for being use in the field of cell and molecular biology. Also, from the jellyfish *Aequorea victoria* the so-called green fusion protein was obtained for the first time. The discovery authors won the Nobel Prize in Chemistry in 2008.

Also, enzymes from marine bacteria have various uses, as extracellular proteases used in detergent and industrial cleaning applications. Other enzymes are resistant to salinity, which confers special features for use them in certain industrial sectors, such as osmosis.

Other marine organisms produce natural substances that protect them against pathogenic substances. Using biotechnology,scientist around the world try to replicate the effects of these substances, as well as try to replicate sticky substances produced by certain mollusks.

Some algae contain collagen, a protein that is part of the organism in many mammalian. In humans conforms the 35% of the total protein. Thus, a type of collagen (collagen VIII) is part of the surrounding layer of the vesicles in the blood, while the skin contains mainly collagen types I and III.

An antifouling has been described by comprising an enzyme obtained from a marine organism and a substrate for this enzyme [MX PA01012448 (A)]. Similarly, a method for specific chitin oligosaccharides production from a marine organism and their applications [MX PA01004251 (A)] has been described, specifically from *Vibrio furnissii.*

Also, the method of obtaining a bioactive extract from a marine organism (*Holothuria scabra*) for being used as a natural fluorescent dye for a cosmetic composition [DE 10117303 (A1)] has been described. Other extracts from marine organisms have also been used in cosmetics [KR 20050078283 (A), FR 2590273 (A1)].

The protein called aquaporin 3 (AQP3) is present in the plasma membrane of keratinocytes of the epidermis (Sougrat et al., J. Invest. Dermatol.2002, 118, 678-85) and plays an important role in controlling the flow of water through the skin. Thus, these aquaporins are able of transporting glycerol which in turns is involved in the formation of the hydro film that keeps the flexibility and sensory qualities of the stratum corneum. Hydration and AQP3 content in keratinocytes are related and an increase of skin AQP3 involves an improvement of the epidermis hydration (Dumas et al. J. Drugs Dermatol. 2007, 6 Suppl, S20- 24).

However, the recent discovery that skin carcinoma cells overexpressing human AQP3 aquaporin (Hara-Chikuma and Verkman, 2008b. Molecular and Cellular Biology, 28, 326-332) suggests caution in the use of modulators of aquaporin expression to promote skin hydration.

An alternative to the use of ingredients able to promote the synthesis of aquaporins is the use of naturally occurring vesicles enriched not only in aquaporins, but also in other membrane proteins that would be used as the lipid vehicle in the release of water or other substances in the epidermis.

In plants, the extraction of membrane vesicles is obtained by successive centrifugation steps or centrifugations (Larsson et al. Methods Enzymol. 1987, 148, 558-568). They separate the membrane fraction from other cellular fractions. To proceed, after the cold tissue homogenization, a separation in dextran/polyethylene glycol should be performed.

Several extraction methods for obtaining lipid fractions from marine and aquatic organisms have been already described. However, these methods use organic solvents such as ketone [JP 2008150586 (A)] or pressurized solvents such as propane, butane or hexane [WO 0023546 (A1)] which may be incompatible with the subsequent skin application of the final product. In addition, these lipid obtained fractions are total lipids, but not complete membrane vesicles. They lack of membrane transport proteins necessary to act as a carrier of certain substances.

### DESCRIPTION OF THE INVENTION

The present invention refers to a procedure for obtaining plant-plasma membrane vesicles, enriched with membrane transport proteins from a marine organism, not explored for this purpose, and in which, once obtained, can be included other products, natural or synthetic, with dermatological, pharmacological, therapeutic or industrial purposes, such as a hygroscopic chemical.

Among any of the industrial purposes, these membrane vesicles can be used, for example, for the manufacture of biomembranes containing them, allowing the selective transport of substances through them, since these vesicles are rich in membrane transport proteins.

The invention is as provided in the claims.

Afirst aspect of the disclosure relates to the extraction procedure and the use of a marine organism, preferably from animals, for obtaining vesicle membrane enriched with transport proteins.

In the present invention, the expression "vesicle enriched with transport proteins" refers to a membrane vesicle of natural origin from a marine organism that belongs to the family *Cnidaria.*

Generally, the method of producing membrane vesicles (also referred to herein as membrane vesicles extraction procedure or procedure of the invention) high in intrinsic membrane proteins from a marine organism comprises:
- cold homogenization of a marine animal tissue by an automatic mechanical homogenizer, wherein the tissue can be a whole marine animal or at least a part of this animal,
- separation of a membrane fraction by successive centrifugations and use of an aqueous two-phase extraction system of dextran/polyethylene glycol,
- re-suspension of the membrane fraction in a preservation and pharmaceutically acceptable buffer.

The vesicle extraction procedure was realized according to the technique of Larsson et al., 1987, which is incorporated in the reference list. However, the methodology included some modifications which make the process suitable for industry, because of the lower costs and shorter time extraction.

Thus, instead of vacuum filtration and manual homogenization it was employed a homogenizer system with automatic mechanical homogenization under cold, polytron type or mechanical mixer (Thermomix), with different combinations of speed and time, in a preferred temperature range of 4 and 15°C, and more preferably between 4 and 10°C, including those limits. Homogenization speed varied from 0 to 5000 rpm, with an initial time-course from 0 to 1 min and a speed of 1000-4000 rpm and a second homogenization step from 0 to 25 s with a speed of 3000-5000 rpm.

Also, the conservation buffer was modified, eliminating those compounds that may result toxic or harmful to human health, such as PIPES buffer, described in the published extraction method, which is absorbed through the skin and was replaced by another acceptable pharmaceutical buffer preferable selected from the following: bicarbonate buffer, phosphate buffer and acetate buffer.

The term "pharmaceutically acceptable buffer" refers to a buffer that is not biologically or otherwise undesirable, and that can be administered to a person or an animal without causing any undesirable biological effects or interacting in a deleterious manner with other products or components contained in the vesicles.

In a preferred procedure, the marine organism is a marine animal, and more preferably a filter feeders marine animal.

This type of filter feeders animals are characterized by their high capacity to transport water and other solutes, due in part to the expression of specific proteins for this purpose, which are useful for the present invention.

For example, and without limitation, the marine animal may be a sponges from a porifers species (Porifera), a filter from echinoderm specie (Echinodermata), a filter from polychaete specie (Polychaeta), a filter from ascidiaceus specie (Ascidiacea), a filter from mollusks specie (Mollusca), a filter from cnidarians specie (Cnidaria), etc., and combinations of them.

In a more preferred realization the filter marine organism is a marine animal belonging to the phylum Cnidaria, and more preferably belonging to the class Scyphozoa (Scyphozoa).

The term phylum (also referred to division in taxonomy) refers to the fundamental taxonomic category of biological classification, which includes organisms by common ancestry and that match a given pattern of organization, such as mollusks, chordates or annelids.

The term class refers to the taxonomic group situated between phylum or division and order, comprising several orders of plants or animals with many common characters.

A second aspect relates to membrane vesicles obtained according to the above methodology, comprising an effective amount of membrane proteins for their use in different fields, and most preferably, for use in cosmetic, pharmacological or therapeutic purposes.

In the present invention, an "effective amount" is referred as the quantity of membrane proteins into vesicles obtained by the process of the invention enough to have efficacy at hydration/health level for both humans and animals.

A third aspect relates to the use of membrane vesicles obtained from a marine organism, since they may be a natural carrier/vehicle for the release/delivery of water or bioactive substances, also they can act as filters.

Within the scope of this disclosure, biofilter, also called biological filters, refers to devices obtained from organic material used to filter out a range of compounds. In this sense, the vesicles of the present invention can be used as biofilters, for filtering water, chemical or biological fluids, and more particularly, for example, as filters for water purification or blood filters for dialysis.

In this sense, an application refers to the use of these membrane vesicles enriched with membrane transporter proteins for commercial exploitation, since they may be a natural medium for water release, bioactive substances, hygroscopic agents, salts or other chemicals substances such as antibiotics or any formulation for cosmetic or therapeutic purposes. Accordingly, one aspect of the invention relates to membrane vesicles of the present invention for use for cosmetic, pharmacological or therapeutic purposes.

In the treatment of a pathophysiological process, it is desirable that the drug delivery was performed such that the drug reaches its site of action at a given concentration. In this line, lipoproteins and glycoproteins, among others, have been used as vectors for drugs, which have the advantage of being biodegradable and endocytable.

Moreover it is widely known that liposomes are microscopic vesicles constituted by concentric phospholipid bilayers with aqueous compartments which are capable of capturing a variety of hygroscopic active substances, liposoluble or amphiphilic.

Therefore, and based on the above, this invention refers to the use of animal-plasma membrane vesicles as transport systems or vectors for specific substances delivery for topical application (creams, shampoos, ointments, serums, tonics) or therapeutic purposes.

These vesicles contain membrane transport proteins in their structure allowing the release of water, ions and other substances such as urea and glycerol (hydration agents) that can be transported through the membrane proteins and therefore employed for cosmetic or dermatological formulations, hydration or stitches cure, burns and other injuries.

Furthermore, the disclosure refers to the use of membrane vesicles containing toxins released by the jellyfish with therapeutic utilities and the extracted collagen from the jellyfish. Also, plant derived bioactive substances (vitamin C, glucosinolates, or phenolic compounds) with antioxidant and/or hydrating capacity could be included to use them in formulations prepared for this purpose.

In other words, another aspect of the invention relates to the use of a vesicle obtained from the above procedure, as a delivery system or vector of at least one further substance (bioactive or pharmaceutical compound), so that they can be applied for cosmetic, pharmacological or therapeutic purposes. In a preferred realization, the use of this vesicle obtained by the process of the present invention is as a delivery system or vector of a toxin from jellyfish with therapeutic utilities.

Furthermore, in another aspect, the invention refers to the vesicles containing, either in their membrane or inside, at least one further substance being applied for cosmetic, pharmacological and/or therapeutic purposes

In an example, without limitation, such additional substances can be a natural bioactive compound (such as a plant-compound with antioxidant and/or hydration properties, such as a vitamin, a protein, a fatty acid, a glucosinolate, a phenolic compound, a naturally-hygroscopic agent- for example urea, glycerol, a toxin from jellyfish, etc.), a synthetic chemical agent (for example a chemical synthetic antibiotic, a synthetic hygroscopic agent, etc.) and a combination of them.

In another aspect, the present disclosure refers to a pharmaceutical or cosmetic formulation comprising an effective acceptable amount of at least one of the marine membrane vesicles enriched with membrane transporter proteins object of the present invention, either containing or no additional substances. It is also an object of this invention, the use of these membrane vesicles for the preparation of a pharmaceutical or cosmetic formulation.

In the present disclosure, "effective acceptable amount of membrane vesicles" refers to the enough amount of membrane vesicles to be effective for the intended use.

Another aspect of the disclosure relates to one of the vesicles of the present invention or a pharmaceutical formulation that contains them, for use in medicine. In a preferred realization the above vesicle or pharmaceutical formulation is used in skin hydration.

### BRIEF DESCRIPTION OF THE FIGURES

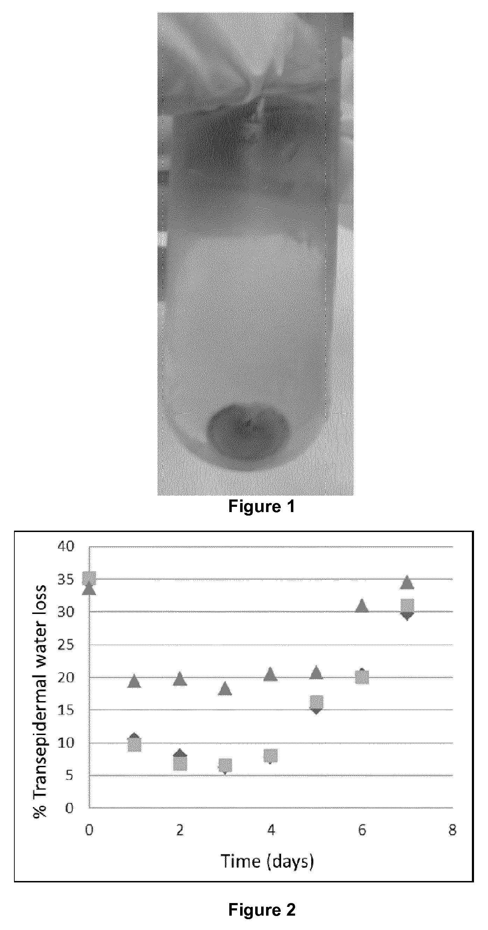

**Fig 1****.** Membrane vesicles resulting from marine organism extraction. Plasma membrane vesicles precipitate obtained by homogenization and centrifugation separation is shown.
**Fig. 2****.** Comparison of the hydrating effect of the vesicles with and without hygroscopic agent. The protein concentration of plasma membrane vesicles present in the application was 2 µg µL⁻¹. ■ Application of the vesicles with hygroscopic agent; ◆ Application of the vesicles without hygroscopic agent; ▲ Direct application of the hygroscopic agent without vesicles.

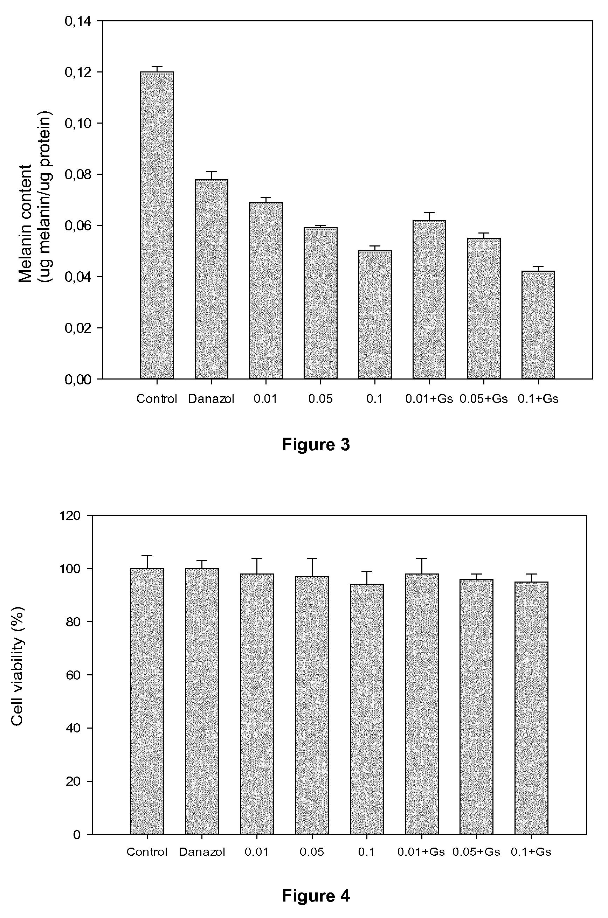

**Fig. 3****.** Melanin content of B16 cells cultivated for 1 day and stimulated with IBMX. Danazol was used as inhibitor of melanogenesis for positive control. The culture medium was supplemented with membrane vesicles obtained from *Scyphozoa* (concentrations of 0.01, 0.05 and 0.1%) and vesicles containing glucosinolates (+Gs) at similar concentrations (n = 3).
**Fig 4****.** Percent survival of B16 cells cultivated for 1 day and stimulated with IBMX. Danazol was used as inhibitor of melanogenesis for positive control. The culture medium was supplemented with membrane vesicles obtained from Scyphozoa (concentrations of 0.01, 0.05 and 0.1%) and vesicles containing glucosinolates (+ Gs) at similar concentrations (n = 3).

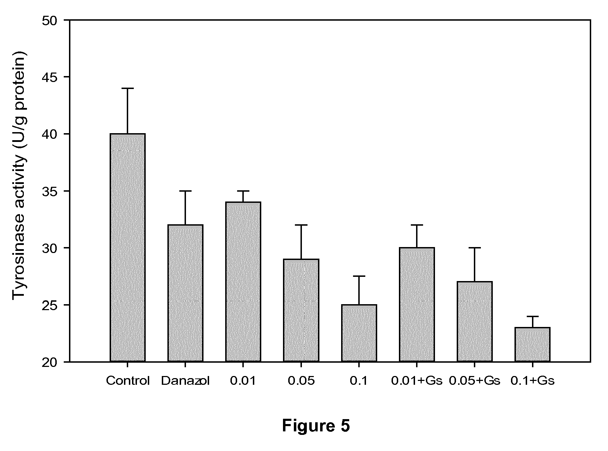

**Fig 5****.** Tyrosinase activity in B16 cells cultured for 1 day and stimulated with IBMX. Danazol was used as inhibitor of melanogenesis for positive control. The culture medium was supplemented with membrane vesicles obtained from *Scyphozoa* (concentrations of 0.01, 0.05 and 0.1%) and vesicles containing glucosinolates (+Gs) at similar concentrations (n = 3).

### EXAMPLES

### EXAMPLE 1: Extraction Procedure membrane proteins of animal origin.

Protein extraction was automatically performed from *Scyphozoa* tissue (20 g) from parts of the umbrella-animal, sub-umbrella and tentacles. This tissue was homogenized using a homogenizer, at 4000 rpm for 15 seconds as an initial stage and at 5000 rpm for 20 seconds as a second step. The tissue sample is homogenized with extraction buffer in a ratio 2/1 of buffer/sample.

The extraction buffer contained 2-[4-(2-hydroxyethyl)-1-piperazinyl] ethanesulfonic acid (HEPES) 50 mM and 0.5 M mannitol (pH 7.5), to this solution was then added dithiothreitol (DTT) 1 mM, 5 mM ascorbic acid and insoluble polyvinylpyrrolidone (PVP) 0.6% (w/v), 10% glycerol and 5 mM β - glycerophosphate.

The homogenate was centrifuged at 10000 g for 20 minutes at 4 ° C. The supernatant was collected and centrifuged at 55000 g for 35 minutes at 4 ° C. The pellet was re-suspended in 2 ml of re-suspension buffer (0.3 M mannitol, 5 mM bicarbonate buffer at pH 7.0-8.0). At this point, the sample is added to the two-phase system and centrifuged at 6000 g at 4 ° C. The upper phase was centrifuged at 55000 g for 35 minutes at 4 ° C (Figure 1 ) and re-suspended in 1ml of final buffer (5mM HEPES - 0.3M sucrose). The yield of plasma membrane protein resulted between 7-10 mg/ml. In the sponge *Suberites domuncula* the yield was 6.6 mg/ml (Müller et al., 1996. Marine Biology 125:165-170).

To incorporate additional substances into the vesicles, it was preceded by adding the additional substance into the vesicles re-suspension at 4°C, at equivalent molarity to the protein concentration vesicles. Subsequently, we proceeded to a stirring 10 seconds at 5000 rpm. Subsequently, it was centrifuged at 55000 g for 35 minutes at 4 ° C.

**Table 1 shows a list of identified membrane proteins by HPLC -MS- MS in the marine organism.**

| **Peptides** | **Mol. W.** | **Specie** | **Protein** |
|---|---|---|---|
| 1 | 29154.6 | *Danio rerio* | Aquaporin 1a |
| 1 | 3909.5 | *Xenopus tropicalis* | RuvB- 1 |
| 1 | 8813.1 | *Caenorhabditis elegans* | Hypothetical protein C52G5.3 |
| 1 | 11915.9 | *Caenorhabditis elegans* | Hypothetical protein F34H10.2 |
| 1 | 15943.2 | *Caenorhabditis elegans* | ComPleXin (synaptic protein) homolog family member (cpx-1) |
| 1 | 16890.3 | *Caenorhabditis elegans* | Hypothetical protein R11 G11.6 |
| 1 | 17195.6 | *Caenorhabditis elegans* | Hypothetical protein T02H6.8 |
| 1 | 19186.9 | *Danio rerio* | Hypothetical protein LOC566423 |
| 1 | 19695.9 | *Caenorhabditis elegans* | Family MLP/CRP (Mollusks LIM Protein/enriched Cysteine) (mlp-1) |
| 1 | 21044.5 | *Danio rerio* | Type A protein 15 basic helix-loop-helix |
| 1 | 21049.8 | *Caenorhabditis elegans* | Hypothetical protein Y54G2A.44 |
| 1 | 21187.8 | *Caenorhabditis elegans* | Hypothetical protein F42F12.4 |
| 1 | 23183.0 | *Caenorhabditis elegans* | Hypothetical protein C34B2.5 |
| 1 | 24135.1 | *Danio rerio* | Component complement C8 gamma chain |
| 1 | 24307.4 | *Caenorhabditis elegans* | Hypothetical protein F33C8.4 |
| 1 | 25206.2 | *Caenorhabditis elegans* | Hypothetical protein B0507.4 |
| 1 | 28841.9 | *Caenorhabditis elegans* | NTH (endonuclease Three like) homolog family member (nth-1) |
| 1 | 31453.6 | *Danio rerio* | ras-related protein Rab-40B |
| 1 | 31763.4 | *Caenorhabditis elegans* | Hypothetical protein F58F9.8 |
| 1 | 33515.7 | *Caenorhabditis elegans* | Hypothetical protein ZK177.2 |
| 1 | 33654.1 | *Caenorhabditis elegans* | Hypothetical protein Y53F4B.36 |
| 1 | 33654.1 | *Caenorhabditis elegans* | Hypothetical protein Y53F4B.36 |
| 1 | 33681.6 | *Xenopus laevis* | Uncoupling protein 3 (mitochondrial, proton carrier) |
| 1 | 34061.4 | *Xenopus laevis* | Hypothetical protein LOC414618 |
| 1 | 34338.7 | *Caenorhabditis elegans* | Hypothetical protein Y71G12B.10 |
| 1 | 35824.5 | *Caenorhabditis elegans* | Hypothetical proteinF53B6.7 |
| 1 | 35888.0 | *Danio rerio* | Voltage-dependent calcium channel gamma-2 subunit |
| 1 | 36212.5 | *Xenopus laevis* | MGC52890 protein |
| 1 | 37273.7 | *Caenorhabditis elegans* | Serpentine Receptor, class E (epsilon) family member (sre-9) |
| 1 | 37744.7 | *Xenopus laevis* | serine/threonine kinase 35 |
| 1 | 38250.7 | *Caenorhabditis elegans* | Serpentine Receptor, class W family member (srw-75) |
| 1 | 39923.3 | *Xenopus laevis* | rab9 effector protein with kelch motifs |
| 1 | 39972.0 | *Caenorhabditis elegans* | Hypothetical protein Y41D4B.11 |
| 1 | 41815.8 | *Caenorhabditis elegans* | Hypothetical protein C13A2.12 |
| 5 | 41838.1 | *Danio rerio* | actin, aortic smooth muscle |
| 1 | 42055.3 | *Danio rerio* | actin, aortic smooth muscle |
| 1 | 43005.6 | *Danio rerio* | Methionine adenosyltransferase-like |
| 1 | 43111.5 | *Caenorhabditis elegans* | Hypothetical protein K09H9.1 |
| 1 | 43731.3 | *Caenorhabditis* | Hypothetical protein C31 B8.4 |
| | | *elegans* | |
| 1 | 44136.3 | *Danio rerio* | Protein like gag |
| 1 | 44229.2 | *Caenorhabditis elegans* | BTB and MATH domain containing family member (bath-34) |
| 1 | 44368.2 | *Caenorhabditis elegans* | Hypothetical protein T25F10.6 |
| 1 | 45370.8 | *Danio rerio* | Homologo LAG1, ceramide synthase 2 |
| 1 | 45388.5 | *Danio rerio* | zinc finger protein AEBP2 |
| 1 | 45456.6 | *Danio rerio* | Protein Tmem184a |
| 1 | 45601.7 | *Caenorhabditis elegans* | Hypothetical proteinF47G6.3 |
| 1 | 45806.5 | *Caenorhabditis elegans* | Hypothetical protein T05H10.6 |
| 1 | 46619.2 | *Caenorhabditis elegans* | Hypothetical protein Y48B6A.5 |
| 1 | 46786.0 | *Danio rerio* | Protein kinase C |
| 1 | 47208.8 | *Danio rerio* | flotilin 1b |
| 1 | 47261.6 | *Caenorhabditis elegans* | Hypothetical protein T24B8.2 |
| 1 | 48733.2 | *Danio rerio* | growth/differentiation factor 9 |
| 1 | 49167.4 | *Danio rerio* | Hypothetical protein LOC678521 |
| 1 | 49736.4 | *Caenorhabditis elegans* | Hypothetical protein Y38E10A.t |
| 4 | 49816.3 | *Xenopus laevis* | Tubulin chain beta-4 |
| 5 | 49826.3 | *Danio rerio* | Beta tubulin Type I |
| 5 | 50180.0 | *Danio rerio* | Tubulin alpha-1B |
| 2 | 50668.6 | *Caenorhabditis elegans* | Elongation factor (eft-3) |
| 1 | 50751.5 | *Danio rerio* | STAR-related lipid transfer protein 3 |
| 1 | 50900.0 | *Caenorhabditis elegans* | Hypothetical protein Y38F2AR.6 |
| 1 | 51793.6 | *Xenopus laevis* | protein MGC84721 |
| 1 | 51807.4 | *Caenorhabditis elegans* | GDI (RabGDP Dissociation Inhibitor) (gdi-1) |
| 1 | 52081.4 | *Xenopus laevis* | LOC733413 protein |
| 1 | 53593.7 | *Caenorhabditis elegans* | Cyclin-Dependent Kinase family member (cdk-9) |
| 1 | 54196.3 | *Danio rerio* | calcium/calmodulin-dependent protein kinase type II delta 1 chain isoform 2 |
| 1 | 55004.7 | *Caenorhabditis elegans* | INneXin family member (inx-21) |
| 1 | 55265.2 | *Danio rerio* | novel zinc finger domain containing protein |
| 1 | 57249.7 | *Caenorhabditis elegans* | Hypothetical protein F57B7.1 |
| 6 | 57527.1 | *Caenorhabditis elegans* | ATP synthase subunit family member (atp-2) |
| 1 | 57654.2 | *Xenopus laevis* | LOC431899 protein |
| 1 | 58338.4 | *Danio rerio* | Protein Qrsl1 |
| 1 | 58471.3 | *Caenorhabditis elegans* | Flavin-containing MonoOxygenase family member (fmo-3) |
| 1 | 58495.6 | *Caenorhabditis elegans* | Hypothetical protein C39B5.6 |
| 1 | 58950.8 | *Caenorhabditis elegans* | Hypothetical protein C46A5.2 |
| 1 | 59229.2 | *Xenopus laevis* | Peptidilprolil isomerasa- 2 |
| 1 | 59888.2 | *Xenopus laevis* | ATP synthase subunit alpha, mitochondrial |
| 1 | 60065.8 | *Xenopus laevis* | Fosfoglucomutase 3 |
| 1 | 60116.1 | *Caenorhabditis elegans* | Hypothetical protein Y51 H4A.25 |
| 1 | 60303.2 | *Danio rerio* | motile sperm domain-containing protein 2 |
| 1 | 60844.6 | *Danio rerio* | Harmonin |
| 1 | 61414.2 | *Caenorhabditis elegans* | Hypothetical protein F28H7.6 |
| 1 | 61892.7 | *Danio rerio* | Epoxide hydrolase 2 |
| 1 | 62425.8 | *Caenorhabditis elegans* | Hypothetical protein T10B11.2 |
| 1 | 64870.6 | *Caenorhabditis elegans* | Protein Phosphatase 2A (Two A) Regulatory subunit family member (pptr-2) |
| 1 | 65552.7 | *Danio rerio* | Protein Aatf |
| 1 | 65772.0 | *Xenopus laevis* | mediator of RNA polymerase II transcription subunit 26 |
| 1 | 66084.3 | *Danio rerio* | Ligatin |
| 1 | 66890.1 | *Danio rerio* | NADPH-dependent diflavin oxidoreductase 1 |
| 1 | 66906.4 | *Caenorhabditis elegans* | WHiTe (Drosophila) related ABC transport family member (wht-1) |
| 1 | 68060.2 | *Danio rerio* | Protein like CG17293-PA- |
| 1 | 68135.9 | *Caenorhabditis elegans* | Hypothetical proteinY104H12D.3 |
| 1 | 69070.9 | *Danio rerio* | cryptochrome 1 b |
| 1 | 69435.3 | *Caenorhabditis elegans* | Hypothetical protein T04G9.6 |
| 1 | 69955.7 | *Caenorhabditis elegans* | Hypothetical protein W03B1.9 |
| 1 | 70484.7 | *Danio rerio* | Cell division cycle 16 |
| 1 | 70638.6 | *Caenorhabditis elegans* | Hypothetical proteinC41C4.3 |
| 1 | 70960.8 | *Xenopus laevis* | Plastin 3 |
| 1 | 71408.2 | *Xenopus laevis* | Protein kinase D1 |
| 1 | 72735.6 | *Xenopus laevis* | Protein MGC83110 |
| 1 | 74788.9 | *Xenopus laevis* | Protein LOC398650 |
| 1 | 74819.8 | *Caenorhabditis elegans* | Hypothetical protein C16A11.5 |
| 1 | 75039.0 | *Caenorhabditis elegans* | Hypothetical protein T05G5.9 |
| 1 | 75223.3 | *Xenopus laevis* | zinc finger and BTB domain containing 5 |
| 1 | 75913.4 | *Danio rerio* | Protein kinase 1cGMP-dependent |
| 1 | 78281.1 | *Caenorhabditis* | NOT-Like (yeast CCR4/NOT |
| | | *elegans* | complex component) family member (ntl-3) |
| 1 | 78421.5 | *Caenorhabditis elegans* | Hypothetical protein T20B6.2 |
| 1 | 78982.8 | *Xenopus laevis* | Protein LOC446929 |
| 1 | 78988.8 | *Caenorhabditis elegans* | Hypothetical protein F14D12.1 |
| 1 | 79973.0 | *Caenorhabditis elegans* | Hypothetical proteinY39G10AR.10 |
| 1 | 80561.3 | *Caenorhabditis elegans* | Hypothetical protein Y75B8A.19 |
| 1 | 81198.6 | *Caenorhabditis elegans* | Hypothetical protein Y41 D4B.4 |
| 1 | 81611.3 | *Danio rerio* | neurofilament, medium polypeptide |
| 1 | 82568.7 | *Caenorhabditis elegans* | Protein B0361.8 |
| 1 | 83151.6 | *Danio rerio* | Protein 30 F-box |
| 1 | 83346.1 | *Danio rerio* | novel protein similar to human and mouse CASK interacting protein 2 (CASKIN2) |
| 1 | 83382.6 | *Xenopus laevis* | B myb-related protein |
| 1 | 83574.9 | *Xenopus tropicalis* | E1A binding protein p300 |
| 1 | 85633.2 | *Danio rerio* | phosphofructokinase, muscle a |
| 1 | 86229.9 | *Danio rerio* | conserved oligomeric Golgi complex subunit 7 |
| 1 | 88406.8 | *Caenorhabditis elegans* | Hypothetical protein T04F8.6 |
| 1 | 88406.8 | *Caenorhabditis elegans* | Hypothetical protein T04F8.6 |
| 1 | 88439.7 | *Xenopus laevis* | transient receptor potential cation channel, subfamily C, member 4 associated protein |
| 1 | 88948.8 | *Caenorhabditis elegans* | Hypothetical protein C37H5.5 |
| 1 | 89494.7 | *Danio rerio* | Hypothetical protein LOC563679 |
| 1 | 94147.3 | *Xenopus laevis* | RNA binding motif protein 12 |
| 1 | 96583.1 | *Danio rerio* | Phospholipase DDHD1 |
| 1 | 99050.6 | *Caenorhabditis elegans* | Hypothetical protein T04C9.1 |
| 1 | 99151.3 | *Danio rerio* | ATP-binding cassette sub-family F member 1 |
| 1 | 99566.4 | *Xenopus laevis* | family with sequence similarity 59, member B |
| 1 | 103471.9 | *Xenopus laevis* | Protein LOC495508 |
| 1 | 103913.2 | *Caenorhabditis elegans* | Hypothetical protein Y71A12B.17 |
| 1 | 106368.5 | *Caenorhabditis elegans* | Anion/Bicarbonate Transporter family member (abts-4) |
| 1 | 108092.1 | *Caenorhabditis elegans* | EAG K+ channel EGL-2 |
| 1 | 108122.0 | *Danio rerio* | solute carrier family 8 (sodium/calcium exchanger), member 1a |
| 1 | 108531.2 | *Caenorhabditis elegans* | Hypothetical protein F56D12.6 |
| 1 | 111666.1 | *Xenopus laevis* | contactin 1 |
| 1 | 112697.8 | *Xenopus laevis* | poli [ADP-ribosa] polymerase 1 |
| 1 | 118921.6 | *Caenorhabditis elegans* | Valyl tRNA Synthetase family member (vrs-2) |
| 1 | 119444.0 | *Danio rerio* | importin-7 |
| 1 | 129275.1 | *Caenorhabditis elegans* | Initiation Factor 4G (eIF4G) family member (ifg-1) |
| 1 | 131465.4 | *Caenorhabditis elegans* | Hypothetical protein F29D11.2 |
| 1 | 133358.2 | *Caenorhabditis elegans* | SPT transcription factor family member (spt-5) |
| 1 | 133819.5 | *Caenorhabditis elegans* | Hypothetical protein F49E2.5 |
| 1 | 134478.0 | *Caenorhabditis* | Na-K-Cl Cotransporter homolog |
| | | *elegans* | family member (nkcc-1) |
| 1 | 137274.6 | *Caenorhabditis elegans* | Hypothetical protein F26D2.10 |
| 1 | 140213.0 | *Caenorhabditis elegans* | Germinal Center Kinase family member (gck-4) |
| 1 | 145710.5 | *Danio rerio* | erbB-3a |
| 1 | 148982.4 | *Xenopus laevis* | GLI family zinc finger 2 |
| 1 | 151064.9 | *Caenorhabditis elegans* | Hypothetical protein H08M01.2 |
| 1 | 160719.4 | *Caenorhabditis elegans* | Hypothetical protein F47E1.2 |
| 1 | 175255.6 | *Danio rerio* | Protein Zgc:63504 |
| 1 | 177863.7 | *Caenorhabditis elegans* | Hypothetical protein T23F2.2 |
| 1 | 180400.4 | *Caenorhabditis elegans* | Nuclear Pore complex Protein family member (npp-10) |
| 1 | 195781.2 | *Caenorhabditis elegans* | PLeXin (plx-2) |
| 1 | 197587.6 | *Caenorhabditis elegans* | Hypothetical protein F08F8.4 |
| 1 | 217742.0 | *Caenorhabditis elegans* | plexin A |
| 1 | 245904.7 | *Caenorhabditis elegans* | Hypothetical protein Y40C5A.3 |
| 1 | 270640.2 | *Danio rerio* | Talin-1 |
| 1 | 306674.1 | *Xenopus laevis* | inositol 1,4,5-triphosphate receptor, type 1 |
| 1 | 403515.1 | *Danio rerio* | Laminin subunit alpha-5 |
| 1 | 492664.7 | *Caenorhabditis elegans* | Hypothetical protein F55F10.1 |

### EXAMPLE 2. Hydration test on skin vesicles.

The assay was performed with 5 volunteers whose skin had moderate levels of water loss. The process was conducted with pure vesicles at concentration of 2 µg µL⁻¹ storage or stock buffer as described in Example 1. A comparative study of vesicles containing a hygroscopic agent and without the hygroscopic agent was performed. The results were compared with a hygroscopic agent. Measurements were carried out every 24 h on the skin of the back of the hand. Treatment stopped on the third day (Figure 2).

### EXAMPLE 3. Effect of vesicles derived from jellyfish on depigmentation. Study with and without glucosinolates on melanogenesis and cell viability of B16 line. Cell viability was determined by MTT assay.

Cells of the cell line with high metastatic ability, B16 (from ATCC, Rockville, MD) were seeded and cultured with DMEM (Dulbecco's Modified Eagle Medium, Invitrogen), pH 7.4, supplemented with 10 %Invitrogen serum fetal bovine), 10 mM HEPES, 100U ml⁻¹ penicillin and 100 mg ml⁻¹ streptomycin. Plated cells were maintained in a wet oven at 37 ° C and 5% CO2. After incubation for 24 hours, the cells were treated with IBMX (Iso-butyl methyl xanthine) 100 µM and different concentrations of vesicles and vesicles containing glucosinolates. After 2 days, the measurements were carried out. Extracts Scyphozoa membrane vesicles (0.01 to 0.05 and 0.1 % expressed as protein concentration), mixed with the glucosinolate -rich extract obtained from seeds of broccoli. Broccoli seeds were used previously washed, disinfected and dried in an oven for 24 hours at 100°C. The seeds were homogenized with 70% methanol (v/v) to obtain a slurry-like mixture. Samples were heated 70°C for 20 min with stirring. Subsequently, it was centrifuged (17,500 xg at 4°C) for 15 min and evaporated with N₂. Finally the sample was re-suspended in ultrapure water and Milli-Q filtered through mesh of 0.45 µm (Millex HV13 of polietersulfano, Millipore). Vesicle mixtures glucosinolates ultracentrifuged to remove remaining glucosinolates not included in vesicles. Vesicles were again re-suspended in the initial volume.

Melanin content was determined at the end of cultivation in the cells washed twice with PBS. The cells were lysed with cold 20 mM (pH 6.8) sodium phosphate buffer, 1% Triton X-100, PMSF (phenylmethylsulfonyl fluoride) and 1 mM EDTA (ethylenediaminetetraacetic acid). After centrifugation at 15.000 xg for 15 min, the melanin of the pellet was dissolved in 1 N NaOH in 20% DMSO (dimethylsulfoxide) for 1 h was determined spectrophotometrically at 490 nm. The results are shown in Figure 3.

Cell viability was measured by the assay and the Trypan blue exclusion assay of lactate dehydrogenase activity (Figure 4).
Measures tyrosinase activity was determined by the degree of oxidation of L - DOPA. The cells were lysed with buffer 20 mM sodium phosphate (pH 6.8), 1 % Triton X - 100, 1 mM PMSF. Tyrosinase was determined after centrifugation at 15,000 xg for 15 min. The concentration of protein and tyrosinase activity was determined in the supernatant. The reaction medium containing 50 mM (pH 6.8), 2.5 mM L -DOPA sodium phosphate and cell extract was incubated for 15 min at 37°C. Dopachrome formation was determined by spectrophotometry at 375 nm (Figure 5).

## Claims

1. Method for obtaining membrane vesicles high in intrinsic membrane proteins from a marine organism, **characterized in that** said vesicles are obtained from a marine organism belonging to the phylum *Cnidaria* and to the class *Scyphozoa,* and wherein said method comprises:
- Cold homogenization of a tissue from a marine animal by an automatic mechanical homogenizer,
- Separating the membrane fraction by successive centrifugation and use of an two-phase system of dextran-polyethylene glycol
- Re-suspending the plasma membrane fraction in a preservation buffer with pharmaceutically acceptable composition.

2. The method of claim 1 wherein the pharmaceutically acceptable buffer is selected conservation within the group consisting of: phosphate buffer, bicarbonate buffer and acetate buffer.

3. Vesicle obtainable by the process described in any one of the preceding claims.

4. Vesicle according to claim 3 wherein it comprises an effective amount of intrinsic membrane intrinsic proteins for use with cosmetic, pharmacological or therapeutic purposes.

5. Vesicle according to one of claims 3 or 4 **characterized by** the incorporation at least one further substance to be used for cosmetic, pharmacological or therapeutic purposes.

6. Vesicle according to claim 5 characterized that the additional substance is selected from a natural bioactive compound, a synthetic chemical agent, and combinations thereof.

7. Vesicle according to claim 6 characterized that the natural bioactive compound is a compound of plant origin with antioxidant, hydrating, bleaching or a combination of these properties.

8. A pharmaceutical composition comprising at least an effective acceptable amount of at least one vesicle of any of claims 4, 5, 6 or 7.

9. Cosmetic composition comprising at least an effective acceptable amount of at least one vesicle of any of claims 3, 4, 5, 6 or 7.

10. The vesicle of any of claims 4, 5, 6 or 7, or the pharmaceutical composition of claim 8 for use in medicine.

11. Use of a vesicle of any of claims 3 or 4 as a transport system or vector of at least one additional substance.

12. Use according to claim 11, wherein the additional substance is selected from a natural bioactive compound, a synthetic chemical agent, and combinations thereof.

13. Use according to claim 12 characterized that the natural bioactive compound is a toxin derived from jellyfish with bleaching or tensoactive properties.

14. Use according to claim 12 **characterized in that** the natural bioactive compound is a plant origin compound with antioxidant, hydrating characteristics or a combination of both.

15. Use of the vesicles of any of claims 3, 4, 5, 6 or 7 for skin hydration.

16. Use of the vesicles of claim 3 as a biofilter.

## Patentansprüche

1. Verfahren zum Gewinnen von Membranvesikeln mit einem hohen Gehalt an intrinischen Membranproteinen von einem marinen Organismus, **dadurch gekennzeichnet, dass** die Vesikel von einem marinen Organismus gewonnen werden, der zum Stamm *Cnidaria* und zur Klasse *Scyphozoa* gehört, und wobei das Verfahren umfasst:
- Kalthomogenisation eines Gewebes von einem marinen Tier durch einen automatischen, mechanischen Homogenisator,
- Trennen der Membranfraktion durch aufeinanderfolgende Zentrifugation und Verwendung eines Zweiphasensystems aus Dextran-Polyethylenglycol,
- Resuspendieren der Plasmamembranfraktion in einem Konservierungspuffer mit pharmazeutisch verträglicher Zusammensetzung.

2. Verfahren nach Anspruch1, wobei es sich bei dem pharmazeutisch verträglichen Puffer um eine ausgewählte Konservierung innerhalb der Gruppe, bestehend aus Phosphatpuffer, Bicarbonatpuffer und Acetatpuffer, handelt.

3. Vesikel, zu gewinnen durch den in einem der vorhergehenden Ansprüche beschriebenen Vorgang.

4. Vesikel gemäß Anspruch 3, wobei sie eine wirksame Menge intrinischer Proteine intrinsischer Membranen umfasst, zur Verwendung für kosmetische, pharmakologische oder therapeutische Zwecke.

5. Vesikel gemäß einem der Ansprüche 3 oder 4, **gekennzeichnet durch** den Einbau von mindestens einer weiteren Substanz, um für kosmetische, pharmakologische oder therapeutische Zwecke verwendet zu werden.

6. Vesikel gemäß Anspruch 5, **dadurch gekennzeichnet, dass** die zusätzliche Substanz aus einer natürlichen bioaktiven Verbindung, einem synthetischen chemischen Mittel und Kombinationen davon ausgewählt ist.

7. Vesikel gemäß Anspruch 6, **dadurch gekennzeichnet, dass** es sich bei der natürlichen bioaktiven Verbindung um eine Verbindung pflanzlichen Ursprungs mit antioxidierender, hydratisierender, bleichender oder einer Kombination dieser Eigenschaften handelt.

8. Pharmazeutische Zusammensetzung, umfassend mindestens eine wirksame verträgliche Menge von mindestens einer Vesikel nach einem der Ansprüche 4, 5, 6 oder 7.

9. Kosmetische Zusammensetzung, umfassend mindestens eine wirksame verträgliche Menge von mindestens einer Vesikel nach einem der Ansprüche 3, 4, 5, 6 oder 7.

10. Vesikel nach einem der Ansprüche 4, 5, 6 oder 7 oder pharmazeutische Zusammensetzung nach Anspruch 8 zur Verwendung in der Medizin.

11. Verwendung einer Vesikel nach einem der Ansprüche 3 oder 4 als Transportsystem oder Vektor von mindestens einer zusätzlichen Substanz.

12. Verwendung gemäß Anspruch 11, wobei die zusätzliche Substanz aus einer natürlichen bioaktiven Verbindung, einem synthetischen chemischen Mittel und Kombinationen davon ausgewählt ist.

13. Verwendung gemäß Anspruch 12, **dadurch gekennzeichnet, dass** es sich bei der natürlichen bioaktiven Verbindung um ein von Quallen abgeleites Toxin mit bleichenden oder die Oberflächenspannung beeinflussenden Eigenschaften handelt.

14. Verwendung gemäß Anspruch 12, **dadurch gekennzeichnet, dass** es sich bei der natürlichen bioaktiven Verbindung um eine Verbindung pflanzlichen Ursprungs mit antioxidierenden, hydratisierenden Eigenschaften oder einer Kombination von beiden handelt.

15. Verwendung der Vesikel nach einem der Ansprüche 3, 4, 5, 6 oder 7 zur Hauthydratation.

16. Verwendung der Vesikel nach Anspruch 3 als Biofilter.

## Revendications

1. Procédé d'obtention de vésicules membranaires riches en protéines membranaires intrinsèques provenant d'un organisme marin, **caractérisé en ce que** lesdites vésicules sont obtenues à partir d'un organisme marin appartenant au phylum *Cnidaria* et à la classe *Scyphozoa,* et ledit procédé comprenant :
- l'homogénéisation à froid d'un tissu provenant d'un animal marin par un homogénéisateur mécanique automatique,
- la séparation de la fraction de membrane par centrifugation successive et utilisation d'un système à deux phases de dextrane-polyéthylène glycol,
- la re-suspension de la fraction de membrane plasmatique dans un tampon de conservation avec une composition pharmaceutiquement acceptable.

2. Procédé selon la revendication 1, dans lequel le tampon de conservation pharmaceutiquement acceptable est choisi dans le groupe constitué par : un tampon de phosphate, un tampon de bicarbonate et un tampon d'acétate.

3. Vésicule pouvant être obtenue par le procédé selon l'une quelconque des revendications précédentes.

4. Vésicule selon la revendication 3, dans laquelle elle comprend une quantité efficace de protéines membranaires intrinsèques pour une utilisation à des fins cosmétiques, pharmacologiques ou thérapeutiques.

5. Vésicule selon l'une des revendications 3 ou 4, **caractérisée par** l'incorporation d'au moins une autre substance à utiliser à des fins cosmétiques, pharmacologiques ou thérapeutiques.

6. Vésicule selon la revendication 5, **caractérisée en ce que** la substance supplémentaire est choisie parmi un composé bioactif naturel, un agent chimique synthétique, et des combinaisons de ceux-ci.

7. Vésicule selon la revendication 6, **caractérisée en ce que** le composé bioactif naturel est un composé d'origine végétale avec des propriétés antioxydantes, hydratantes, blanchissantes ou une combinaison de ces propriétés.

8. Composition pharmaceutique comprenant au moins une quantité acceptable efficace d'au moins une vésicule selon l'une des revendications 4, 5, 6 ou 7.

9. Composition cosmétique comprenant au moins une quantité acceptable efficace d'au moins une vésicule selon l'une des revendications 3, 4, 5, 6 ou 7.

10. Vésicule selon l'une des revendications 4, 5, 6 ou 7 ou composition pharmaceutique selon la revendication 8 pour une utilisation en médecine.

11. Utilisation d'une vésicule selon l'une des revendications 3 ou 4 en tant que système de transport ou vecteur d'au moins une substance supplémentaire.

12. Utilisation selon la revendication 11, dans laquelle la substance supplémentaire est choisie parmi un composé bioactif naturel, un agent chimique synthétique, et des combinaisons de ceux-ci.

13. Utilisation selon la revendication 12, **caractérisée en ce que** le composé bioactif naturel est une toxine dérivée de méduse avec des propriétés blanchissantes ou tensioactives.

14. Utilisation selon la revendication 12, **caractérisée en ce que** le composé bioactif naturel est un composé d'origine végétale avec des caractéristiques antioxydantes ou hydrantes, ou une combinaison des deux.

15. Utilisation des vésicules selon l'une quelconque des revendications 3, 4, 5, 6 ou 7 pour l'hydratation de la peau.

16. Utilisation des vésicules selon la revendication 3 en tant que biofiltre.
